# EUROPEAN PATENT SPECIFICATION

(11) **EP 0 957 933 B1**
(45) Date of publication and mention of the grant of the patent: **07.05.2008**
(21) Application number: 97940062.9
(22) Date of filing: 31.07.1997
(51) Int. Cl.: A61K 39/015, A61K 39/002, A61K 39/29, A61K 39/39

(54) **VACCINE COMPOSITION AGAINST MALARIA**
VAKZINZUSAMMENSETZUNG GEGEN MALARIA
COMPOSITION VACCINALE CONTRE LE PALUDISME

(30) Priority: 02.08.1996 GB 9616351
(43) Date of publication of application: 24.11.1999
(60) Divisional application: 05077131.0
(73) Proprietor: GlaxoSmithKline Biologicals s.a., 1330 Rixensart (BE)
(72) Inventor: COHEN, Joseph, SmithKline Beecham Biol. S.A., 1330 Rixensart (BE)
(74) Representative: Dalton, Marcus Jonathan William
(86) International application number: PCT/EP1997/004326
(87) International publication number: WO 1998/005355

(56) References cited:
- WO-A-86/05790
- WO-A-93/10152
- WO-A-94/00153
- WO-A-95/17209
- WO-A-95/21192
- WO-A-97/30158
- WO-A-97/30159
- US-A- 4 837 016
- HEPPNER D G ET AL: "Safety, immunogenicity, and efficacy of Plasmodium falciparum repeatless circumsporozoite protein vaccine encapsulated in liposomes." JOURNAL OF INFECTIOUS DISEASES 174 (2). AUGUST 1996. 361-366. ISSN: 0022-1899, XP002048585
- SCHOEDEL F ET AL: "Immunity to malaria elicited by hybrid hepatitis B virus core particles carrying circumsporozoite protein epitopes." JOURNAL OF EXPERIMENTAL MEDICINE 180 (3). 1994. 1037-1046. ISSN: 0022-1007, XP002048586

## Description

The present invention relates to a novel vaccine composition and to its use in medicine, particularly in the prevention of malaria infections.

Malaria, is one of the world's major health problems with 2 to 4 million people dying from the disease each year. One of the most acute forms of the disease is caused by the protozoan parasite, Plasmodium falciparum which is responsible for most of the mortality attributable to Malaria.

The life cycle of P. falciparum is complex, requiring two hosts, man and mosquito for completion. The infection of man is initiated by the inoculation of sporozoites in the saliva of an infected mosquito. The sporozoites migrate to the liver and there infect hepatocytes where they differentiate, via the exoerythrocytic intracellular stage, into the merozoite stage which infects red blood cells (RBC) to initiate cyclical replication in the asexual blood stage. The cycle is completed by the differentiation of a number of merozoites in the RBC into sexual stage gametocytes which are ingested by the mosquito, where they develop through a series of stages in the midgut to produce sporozoites which migrate to the salivary gland.

The sporozoite stage of P. falciparum has been identified as a potential target of a malaria vaccine. The major surface protein of the sporozoite is known as circumsporozoite protein (CS Protein). This protein from strain 7G8 has been cloned, expressed and sequenced (Dame et al Science 225 (1984) p593). The protein from strain 7G8 is characterised by having a central immunodominant repeat region comprising a tetrapeptide Asn-Ala-Asn-Pro repeated 37 times but interspersed with four minor repeats Asn-Val-Asp-Pro. In other strains the number of major and minor repeats vary as well as their relative position. This central portion is flanked by an N and C terminal portion composed of non-repetitive amino acid sequences designated as the repeatless portion of the CS protein.

It has been shown that irradiated sporozoites can provide significant protection against experimental human malaria (Am. J. Trop. Med. Hyg. 24: 297-402, 1975). However, production difficulties makes the use of irradiated sporozoite impractical from the point of view of producing a vaccine.

Several groups have proposed subunit vaccines based on the circumsporozoite protein. Several of these vaccines have undergone clinical testing; one is a synthetic peptide, the other is a recombinant protein (Ballou et al Lancet: i 1277 (1987) and Herrington et al Nature 328:257 (1987).

These vaccines were successful in stimulating an anti-sporozoite response. Nonetheless, the magnitude of the response was disappointing, with some vaccinees not making a response at all. Furthermore, the absence of "boosting" of antibody levels on subsequent injections and results of in vitro lymphocyte proliferation assays suggested that T-cells of most of these volunteers did not recognise the immuno-dominant repeat. Nonetheless, one vaccinee in each study did not develop parasitemia.

International Patent Application No. WO 93/10152 (Smithkline Beecham Biologicals) describes and claims a hybrid protein comprising substantially all the C-terminal portion of the CS protein, four or more tandem repeats of the immunodominant region, and the surface antigen from Hepatitis B virus (HBsAg). Preferably the hybrid protein comprises a sequence which contains at least 160 amino acids which is substantially homologous to the C-terminal portion of the CS protein. The CS protein may be devoid of the last 12 amino-acids from the C terminal.

In particular there is described a protein which comprises a portion of the CS protein of P. falciparum substantially as corresponding to amino acids 210-398 of P. falciparum 7G8 fused in frame via a linear linker to the N-terminal of HBsAg. The linker may comprise a portion of preS2 from HBsAg.

A particular embodiment described in WO 93/10152 is the hybrid protein designated RTS. This hybrid consists of:
**°** A methionine-residue, encoded by nucleotides 1059 to 1061, derived from the Sacchromyes cerevisiae TDH3 gene sequence (nucleotides 1-1058 in this reading frame make up the TDH3 promoter itself). (Musti A.M.et al Gene 1983 25 133-143.
**°** Three amino acids. Met Ala Pro, derived from a nucleotide sequence (1062 to 1070) created by the cloning procedure used to construct the hybrid gene.
**°** A stretch of 189 amino acids, encoded by nucleotides 1071 to 1637 representing amino acids 210 to 398 of the circumsporozoite protein (CSP) of Plasmodium falciparum strain 7G8 (Dame et al supra).
**°** An amino acid (Arg) encoded by nucleotides 1638 to 1640, created by the cloning procedure used to construct the hybrid gene.
° Four amino acids, Pro Val Thr Asn, encoded by nucleotides 1641 to 1652, and representing the four carboxy terminal residues of the hepatitis B virus (adw serotype) preS2 protein (9).
**°** A stretch of 226 amino acids, encoded by nucleotides 1653 to 2330, and specifying the S protein of hepatitis B virus (adw serotype).

WO 93/10152 further describes the expression of the hybrid protein in a recipient yeast strain which already carries in its genome several integrated copies of an hepatitis B S expression cassette. The resulting strain synthesises two polypeptides, S and RTS (or other hybrid protein of the invention), that spontaneously co-assemble into mixed (for example RTS, S) lipoprotein particles. These particles, advantageously present the CSP sequences of the hybrid at their surface.

It is an object of the present invention to confer immunity against P. falciparum and/or P. vivax infestations by immunization with a composition comprising a plurality of antigens in combination with an adjuvant which is a preferential stimulator of TH1 cell response.

Accordingly, the present invention provides a vaccine composition for use in the prevention or treatment of malaria, comprising at least two malaria-derived antigens in combination with an adjuvant which is a preferential stimulator of TH I cell response, and wherein each of the at least two malaria antigens are selected from one of the groups:
a) a hybrid protein comprising substantially all the C-terminal portion of the CS (circumsporozoite) protein, four or more tandem repeats of the immunodominant region, and the surface antigen from Hepatitis B virus (HBsAg);
b) the TRAP of a cloned isolate of P.falciparum from Thailand known as T/9/96, and immunogenic proteins having at least 80% homology thereto; and
c) the MSP-1 of P.falciparum or P.vivax and immunogenic proteins having at least 80% homology thereto, and immunogenic fragments thereof.

Preferably, at least one of the antigens is a hybrid protein as defined above.

A further aspect of the invention provides a vaccine composition for use in the prevention or treatment of malaria, comprising malaria-derived antigens, characterised in that at least one of the antigens is a hybrid protein as defined above.

The amount of antigen present in each vaccine dose is selected as an amount which induces an immunoprotective response without significant, adverse side effects in typical vaccines. Such amount will vary depending upon which specific immunogens are employed. Generally, it is expected that each dose will comprise a total of 1-1000µg of protein, preferably 1-200 µg most preferably 10-100µg. An optimal amount for a particular vaccine can be ascertained by standard studies involving observation of immune responses in subjects. Following an initial vaccination. subjects will preferably receive a boost in about 4 weeks, followed by repeated boosts every six months for as long as a risk of infection exists.

Adjuvants which are capable of preferential stimulation of the TH l cell response are described in International Patent Application Nos. WO 94/00153 and WO 95/17209.

A particular preferred adjuvant comprises QS21, an Hplc purified non-toxic fraction derived from the bark of Quillaja Saponaria Molina, and 3 De-O-acylated monophosphoryl lipid A (3 D-MPL), optionally together with an oil in water emulsion.

3 De-O-acylated monophosphoryl lipid A is known from GB 2220211 (Ribi). Chemically it is a mixture of 3 De-O-acylated monophosphoryl lipid A with 4, 5 or 6 acylated chains and is manufactured by Ribi Immunochem Montana. A preferred form of 3 De-O-acylated monophosphoryl lipid A is disclosed in International Patent Application No. 92/116556.

QS21 is a Hplc purified non toxic fraction of a saponin from the bark of the South American tree Quillaja Saponaria Molina and its method of its production is disclosed (as QA21) in US patent No. 5,057,540.

A preferred oil-in-water emulsion comprises a metabolisible oil, such as squalene, alpha tocopherol and tween 80. Additionally the oil in water emulsion may contain span 85 and/or lecithin.

The ratio of QS21 : 3D-MPL will typically be in the order of 1 : 10 to 10 : 1; preferably 1 : 5 to 5 : 1 and often substantially 1 : 1. The preferred range for optimal synergy is 2.5:1 to 1:1 3D MPL: QS21. Typically for human administration QS21 and 3D MPL will be present in a vaccine in the range 1 µg - 200 µg, such as 1 - 100µg, preferably 10 µg - 50 µg per dose. Typically the oil in water will comprise from 2 to 10% squalene, from 2 to 10% alpha tocopherol and from 0.3 to 3% tween 80. Preferably the ratio of squalene: alpha tocopherol is equal or less than 1 as this provides a more stable emulsion. Span 85 may also be present at a level of 1%. In some cases it may be advantageous that the vaccines of the present invention will further contain a stabiliser.

Vaccine preparation is generally described in New Trends and Developments in Vaccines, edited by Voller et al., University Park Press, Baltimore, Maryland, U.S.A. 1978. Encapsulation within liposomes is described, for example, by Fullerton, U.S. Patent 4.235,877. Conjugation of proteins to macromolecules is disclosed, for example, by Likhite, U.S. Patent 4,372,945 and by Armor et al., U.S. Patent 4,474,757.

Additional malaria-derived antigens useful in the present invention may be selected from the following:
1. A hybrid protein as defined above, such as RTS, more preferably in the form of mixed particles as defined above, such as RTS,S.
2. The TRAP of a cloned isolate of *P. falciparum* from Thailand known as T/9/96, and proteins having at least 80% homology thereto, and immunogenic derivatives including fragments thereof (described in International Patent Application Nos. WO 90/01496 and WO 91/11516 (3i Exploitation Limited), and WO 92/11868 (US Navy)).
3. The 16kD protein described in International Patent Application No.WO 91/18922, and proteins having at least 80% homology thereto, and immunogenic derivatives including fragments thereof.
4. The apical membrane antigen- (AMA-1) of P. falciparum or P. vivax, and proteins having at least 80% homology thereto, and immunogenic derivatives including fragments thereof.
5. The circumsporozoite protein (csp) of P. falciparum or P. vivax, and proteins having at least 80% homology thereto, and immunogenic derivatives including fragments thereof.
6. The MSP-1 of P. falciparum or P. vivax (US Patent No 4,837,016), and proteins having at least 80% homology thereto, and immunogenic derivatives including fragments thereof.
7. Other exoerythrocytic stage proteins and immunogenic derivatives including fragments thereof.
8. Optionally, blood stage proteins and immunogenic derivatives including fragments thereof

The term "immunogenic derivative" encompasses any molecule such as a truncated or other derivative of the protein which retains the ability to induce an immune response to the protein following internal administration to a human. Such other derivatives can be prepared by the addition, deletion, substitution, or rearrangement of amino acids or by chemical modifications thereof.

Immunogenic fragments of the protein, which may be useful in the preparation of subunit vaccines, may be prepared by expression of the appropriate gene fragments or by peptide synthesis, for example using the Merrifield synthesis (The Peptides, Vol 2., Academic Press, NY, page 3).

The immunogenic derivative can be a hybrid, that is, a fusion polypeptide containing additional sequences which can carry one or more epitopes for other Plasmodium immunogens, or other non-Plasmodium immunogens. Alternatively, the immunogenic derivative of the invention can be fused to a carrier polypeptide such Hepatitis B surface or core antigen or to another carrier which has immunostimulating properties, as in the case of an adjuvant, or which otherwise enhances the immune response to the protein or derivative thereof, or which is useful in expressing, purifying or formulating the protein or derivative thereof.

The proteins or immunogenic derivatives thereof which are useful in the invention may be chemically conjugated to a macromolecule using a conventional linking agent such as glutaraldehyde (Geerlings et al, (1988) J, Immunol. Methods, 106, 239-244).

The following Example illustrates the invention:

### Example

### Construction and expression of a recombinant TRAP.

This was prepared as described in WO 90/01496.

### Construction and expression of RTS,S.

This was prepared as described in WO 93/10152.

### Adjuvantation

Two adjuvant formulations were made each comprising the following oil in water emulsion component.
SB26: 5% squalene 5% tocopherol 0.4% tween 80; the particle size was 500 nm size
SB62: 5% Squalene 5% tocopherol 2.0% tween 80; the particle size was 180 nm

### Preparation of emulsion SB62 (2 fold concentrate)

Tween 80 is dissolved in phosphate buffered saline (PBS) to give a 2% solution in the PBS. To provide 100 ml two fold concentrate emulsion 5g of DL alpha tocopherol and 5ml of squalene are vortexed to mix thoroughly. 90ml of PBS/Tween solution is added and mixed thoroughly. The resulting emulsion is then passed through a syringe and finally microfluidised by using an M110S microfluidies machine. The resulting oil droplets have a size of approximately 180 nm.

### Preparation of emulsion SB26

This emulsion was prepared in an analogous manner utilising 0.4% tween 80.

Other emulsions as depicted in the Table were made in an analogous manner.

To each 100ml of emulsion were added the two antigens (10mg of each antigen, equivalent to 50µg per dose) and mixed. This was combined with 100µg/ml of 3D-MPL and 1 00µg/ml of QS21 to give the final formulation. Buffer was set according to salt content and pH.

**Table**

| **Vehicles two fold concentrated** | | | | | | |
|---|---|---|---|---|---|---|
| Emulsions SB | Tocopherol % | Squalene % | Tween 80 % | Span 85 % | Lecithin % | Size |
| 26 | 5 | 5 | 0.4 | 0 | 0 | 500 nm |
| | | | | | | 90-100% |
| | | | | | | 800 nm |
| | | | | | | 10-0% |
| 26.1 | 5 | 5 | 0.4 | 0 | 0.1 | 500 nm |
| 63 | 5 | 5 | 0.6 | 0 | 0 | 500 nm |
| 64 | 5 | 5 | 0.8 | 0 | 0 | 500nm |
| 61 | 5 | 5 | 1 | 0 | 0 | 250-300 mn |
| 62 | 5 | 5 | 2 | 0 | 0 | 180 nm |
| 40 | 5 | 5 | 0.4 | 1 | 0 | 500 mn |
| | | | | | | 80-100% |
| | | | | | | 800 nm |
| | | | | | | 20-0% |
| 40.1 | 5 | 5 | 0.4 | 1 | 0.1 | 500 nm |
| 60 | 5 | 5 | 1 | 1 | 0 | 300 nm |
| 65 | 5 | 5 | 0.4 | 1.5 | 0 | 500 nm |
| 66 | 5 | 5 | 0.4 | 2 | 0 | 500 nm |

### Reference Example Various formulations of RTS,S

RTS,S is described in International patent application No. WO 93/10152 and was formulated for vaccination of balb/c mice. Five animals were in each group. 7 groups of animals received the following formulations
- Group 1: RTS,S SB62
- Group 2: RTS,S QS21 3D-MPL
- Group 3: RTS,S QS21 3D-MPL SB62
- Group 4: RTS,S 3D-MPL Al(OH)₃
- Group 5: RTS,S Al(OH)₃
- Group 6: Plain
- Group 7: Negative control

### (RTS,S-5µg/dose,3 D-MPL 5µg/dose QS21 5µg/dose)

The animals were inoculated and bled at 15 days post first immunisation and at day 7 and 15 post second immunisation and assayed for anti HBSAg antibody subtype. The emulsion SB62 when formulated with QS21 and 3D-MPL enhanced preferentially and in a synergistic fashion the IgG2a antibody response compared to SB 62 alone.

Enhanced IgG2a antibody response in mice is a measure of the ability of the adjuvant system to stimulate a TH1 type response.

## Claims

1. A vaccine composition comprising at least two malarial derived antigens in combination with an adjuvant which is a preferential stimulator of a TH1 cell response, and wherein each of the at least two malaria antigens are selected from one of the groups:
a) a hybrid protein comprising substantially all the C-terminal portion of the CS (circumsporozoite) protein, four or more tandem repeats of the immunodominant region, and the surface antigen from Hepatitis B virus (HBsAg);
b) the TRAP of a cloned isolate of P.falciparum from Thailand known as T/9/96, and proteins having at least 80% homology thereto;
c) the MSP-1 of P.falciparum or P.vivax and immunogenic proteins having at least 80% homology thereto, and immunogenic fragments thereof.

2. A vaccine composition according to claim 1, wherein the adjuvant comprises 3D-MPL.

3. A vaccine composition according to claim 1 or 2, wherein the adjuvant comprises QS21.

4. A vaccine composition according to any one of the preceding claims, wherein the adjuvant comprises an oil-in-water emulsion.

5. A vaccine composition according to any one of the preceding claims, for use in therapy.

6. A vaccine composition according to any one of the preceding claims wherein the hybrid protein a) is the hybrid protein designated RTS.

7. A vaccine composition according to claim 6 wherein the hybrid protein is present in the form of mixed particles designated RTS,S.

8. Use of a vaccine composition according to any one of claims 1 to 7 in the manufacture of a medicament for use in the treatment or prophylaxis of malaria.

## Patentansprüche

1. Impfstoffzusammensetzung, die wenigstens zwei aus Malaria stammende Antigene in Kombination mit einem Hilfsstoff umfaßt, der ein bevorzugter Stimulator einer TH1-Zell-Reaktion ist, und worin jedes der wenigstens zwei Malaria-Antigene aus einer der folgenden Gruppen ausgewählt ist:
(a) ein Hybridprotein, das im wesentlichen den gesamten C-terminalen Teil des CS (Circumsporozoit)-Proteins, vier oder mehr Tandem-Repeats der immundominanten Region und das Oberflächenantigen aus dem Hepatitis B-Virus (HBsAg) umfaßt;
(b) das TRAP eines clonierten Isolats von P. falciparum aus Thailand, bekannt als T/9/96, und Proteine mit wenigstens 80 % Homologie dazu;
(c) das MSP-1 von P. falciparum oder P. vivax und immunogene Proteine mit wenigstens 80 % Homologie dazu und immunogene Fragmente davon.

2. Impfstoffzusammensetzung gemäß Anspruch 1, worin der Hilfsstoff 3D-MPL umfaßt.

3. Impfstoffzusammensetzung gemäß Anspruch 1 oder 2, worin der Hilfsstoff QS21 umfaßt.

4. Impfstoffzusammensetzung gemäß einem der vorhergehenden Ansprüche, worin der Hilfsstoff eine Öl-in-Wasser-Emulsion umfaßt.

5. Impfstoffzusammensetzung gemäß einem der vorhergehenden Ansprüche zur Verwendung in der Therapie.

6. Impfstoffzusammensetzung gemäß einem der vorhergehenden Ansprüche, worin das Hybridprotein (a) das als RTS bezeichnete Hybridprotein ist.

7. Impfstoffzusammensetzung gemäß Anspruch 6, worin das Hybridprotein in Form von als RTS,S bezeichneten gemischten Partikeln vorhanden ist.

8. Verwendung einer Impfstoffzusammensetzung gemäß einem der Ansprüche 1 bis 7 in der Herstellung eines Medikaments zur Verwendung in der Behandlung oder Prophylaxe von Malaria.

## Revendications

1. Composition vaccinale comprenant au moins deux antigènes dérivés du paludisme en association avec un adjuvant qui est un stimulateur préférentiel de la réponse cellulaire TH1 et dans lequel chacun desdits au moins deux antigènes du paludisme est sélectionné dans l'un des groupes suivants :
a) une protéine hybride comprenant de façon substantielle toute la portion C-terminale de la protéine CS (circumsporozoïte), quatre ou plusieurs répétitions en tandem de la région immunodominante, et l'antigène de surface provenant du virus de l'hépatite B (HBsAg) ;
b) la protéine TRAP d'un isolat cloné de *P. falciparum* provenant de Thaïlande connu comme T/9/96, et des protéines immunogènes présentant au moins 80 % d'homologie avec celle-ci;
c) la protéine MSP-1 de *P. falciparum* ou de *P. vivax* et des protéines immunogènes présentant au moins 80 % d'homologie avec celles-ci et des fragments immunogènes de celles-ci.

2. Composition vaccinale selon la revendication 1, dans laquelle l'adjuvant comprend le 3D-MPL.

3. Composition vaccinale selon la revendication 1 ou 2, dans laquelle l'adjuvant comprend le QS21.

4. Composition vaccinale selon l'une quelconque des revendications précédentes, dans laquelle l'adjuvant comprend une émulsion huile dans l'eau.

5. Composition vaccinale selon l'une quelconque des revendications précédentes pour une utilisation en thérapie.

6. Composition vaccinale selon l'une quelconque des revendications précédentes dans laquelle la protéine hybride a) est la protéine hybride dénommée RTS.

7. Composition vaccinale selon la revendication 6 dans laquelle la protéine hybride est présente sous la forme de particules mélangées dénommées RTS,S.

8. Utilisation d'une composition vaccinale selon l'une quelconque des revendications 1 à 7 pour la fabrication d'un médicament pour une utilisation dans le traitement ou la prophylaxie du paludisme.
